(19) Europäisches Patentamt
European Patent Office
Office européen des brevets

(11) Publication number: **0 275 971 A1**

(12) **EUROPEAN PATENT APPLICATION**

(21) Application number: **88100683.7**

(22) Date of filing: **19.01.88**

(51) Int. Cl.[4]: **C07D 401/04**, C07D 207/14

(30) Priority: **21.01.87 JP 11907/87**

(43) Date of publication of application: **27.07.88 Bulletin 88/30**

(84) Designated Contracting States: **BE CH DE ES FR GB IT LI NL SE**

(71) Applicant: **KYORIN PHARMACEUTICAL CO., LTD.**
**No. 5, Kanda Surugadai 2-chome**
**Chiyoda-ku Tokyo(JP)**

(72) Inventor: **Irikura, Tsutomu**
**No. 10-28, Ooizumigakuen-cho 7-chome**
**Nerima-ku Tokyo(JP)**
Inventor: **Suzue, Seigo**
**No. 13-4, Aoba 4-chome**
**Kuki-shi Saitama-ken(JP)**
Inventor: **Murayama, Satoshi**
**No. 6095, Tomonuma**
**Shimotsuga-gun Tochigi-ken(JP)**
Inventor: **Hirai, Keiji**
**No. 1-2-512, Aoba 1-chome**
**Kuki-shi Saitama-ken(JP)**
Inventor: **Ishizaki, Tokayoshi**
**No. 9-6, Sakurada 4-chome Washimiya-machi**
**Kitakatsushiga-gun Saitama-ken(JP)**

(74) Representative: **Patentanwälte TER MEER - MÜLLER - STEINMEISTER**
**Mauerkircherstrasse 45**
**D-8000 München 80(DE)**

(54) **Process and intermediates for quinolonecaboxylic acid.**

(57) The present invention is concerned with a certain novel process for the preparation of a quinolonecarboxylic acid of the following formula;

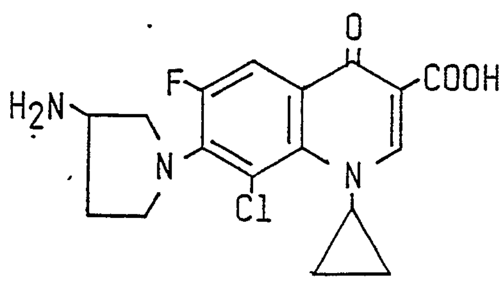

and with intermediates of the following formula;

EP 0 275 971 A1

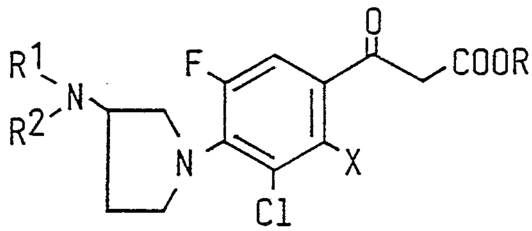

wherein R is hydrogen atom or lower alkyl group, X is halogen atom, $R^1$ and $R^2$ each defined independently are hydrogen atom or a protective group for the amino group, or $R^1$ together with $R^2$ is a protective group, and

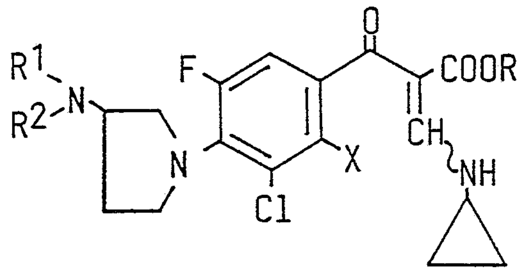

wherein R, $R^1$, $R^2$ and X are as defined above.

# PROCESS AND INTERMEDIATES FOR QUINOLONECARBOXYLIC ACID

Detailed description of the invention

The present invention is related to an improved process for the preparation of a compound of the formula (I):

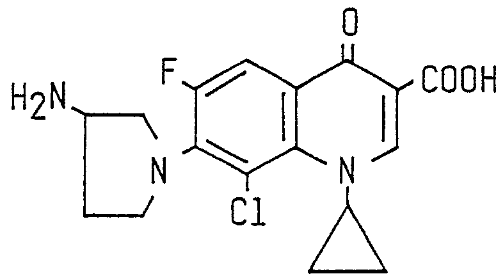

(I)

JP Laid-Open Patent Appl. 86/255181 describes a process for the preparation of a quinolonecarboxylic acid of the formula (I), which comprises treating an intermediate (VI) with a pyrrolidine derivative as the following scheme;

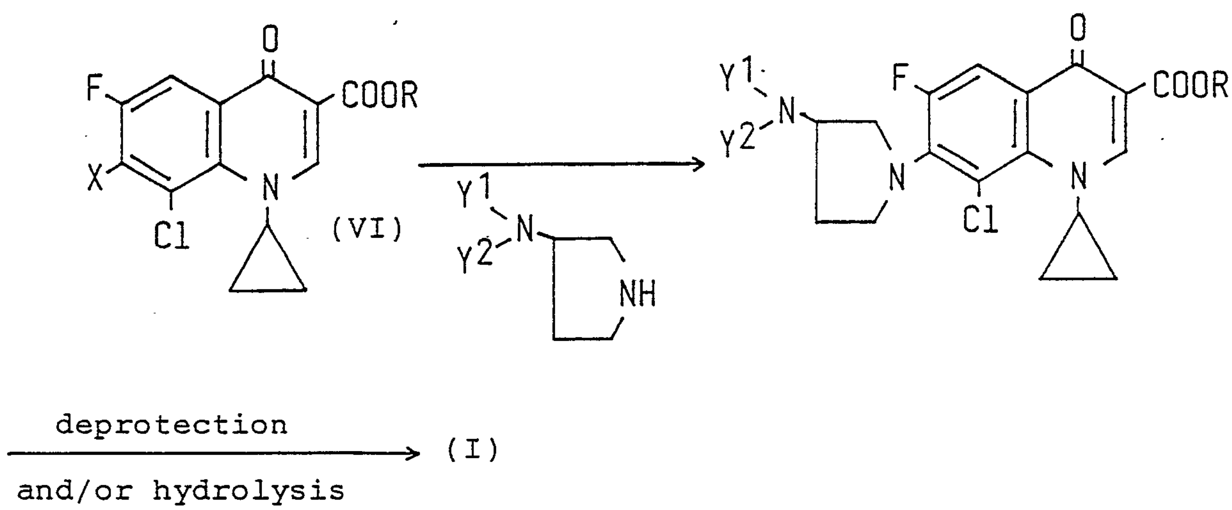


(VI)

(I)

wherein R is hydrogen atom or lower alkyl group, X is halogen atom, $Y^1$ and $Y^2$ each defined independently are hydrogen atom or an amino protective group or $Y^1$ together with $Y^2$ is a protective group.

The above mentioned preparation of the 8-chloro-6-fluoro-7-substituted amino-quinolonecarboxylic acid was carried out by the usual manner such as condensation of a 8-chloro-6, 7-difluoro-quinolonecarboxylic acid derivative with substituted amine. In this procedure, a decrease of purity of the product was caused by the adverse reaction in which reactivity of the 6-position with the amine derivative gave 6-amino substituted isomer (VII) which had no antibacterial activity. The purification process for eliminating the isomer resulted in decrease of the yield.

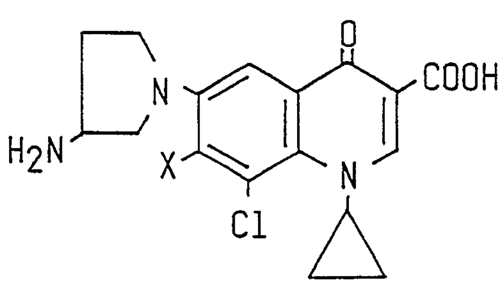

(VII)

On our study of nucleophylic substitution of the 2,3,4,5-tetrafluoro-1-nitrobenzene with substituted cyclic amines, it was found that highly site-selective substitution occurred, especially in a polar solvent on the para-position for electron withdrawing group (e.g., nitro group) and reported in Nippon Kagaku Kaishi, 10, 2054 (1985).

Our continuous study on the basis of the above mentioned information, resulted in an improved process for the compound of the formula (I) in higher quality and excellent yield, which is shown as the following - scheme.

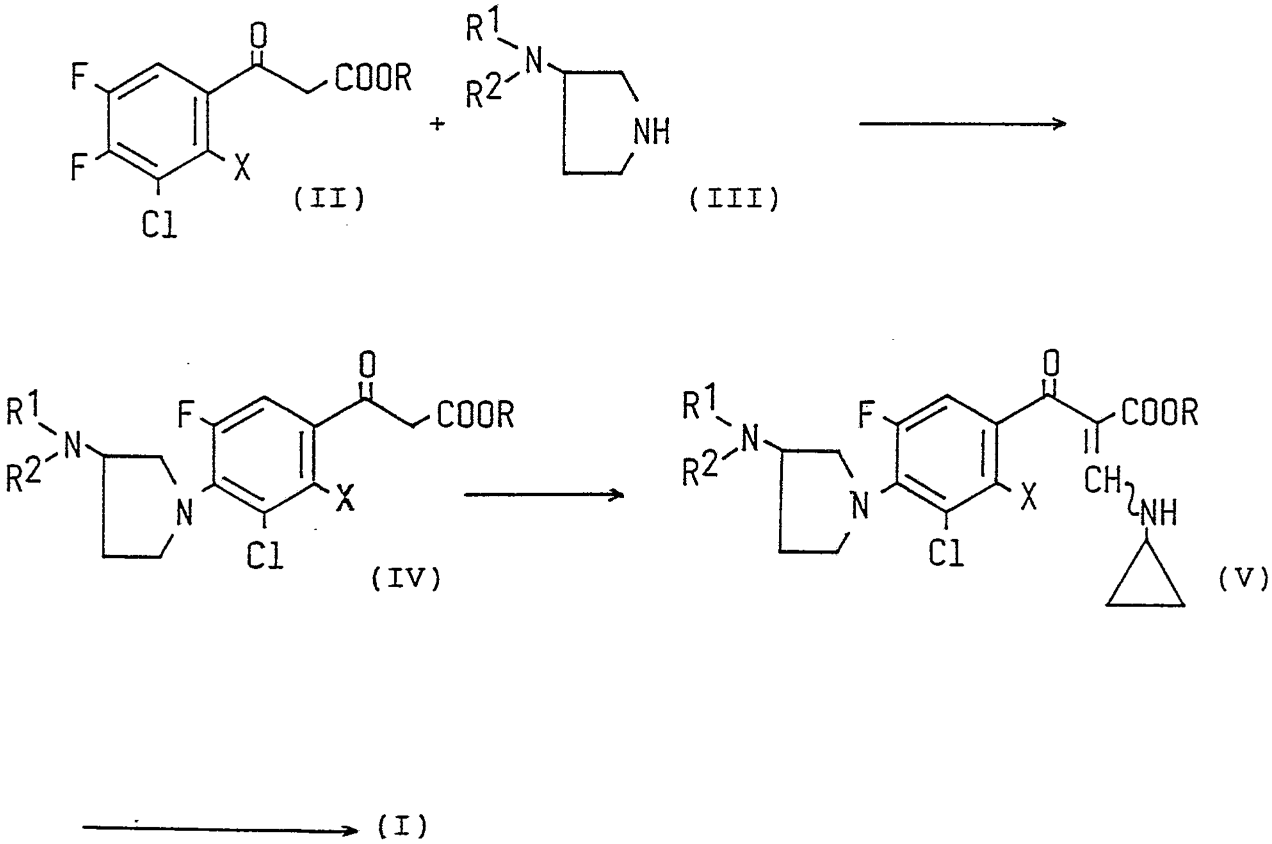

(II) (III) (IV) (V) (I)

A p-(substituted pyrrolidinyl)benzoylacetic acid derivative (IV) is prepared by reacting a benzoylacetic acid derivative (II) with a substituted pyrrolidine (III), wherein R is hydrogen atom or lower alkyl group X is halogen atom, preferably, fluorine atom or chlorine atom, and $R^1$ and $R^2$ are each independently hydrogen atom, lower acyl group such as, e.g., acetyl, propionyl and butyryl group, alkoxycarbonyl group such as, e.g., ethoxycarbonyl, t-butoxycarbonyl or substituted or non-substituted benzyloxycarbonyl group, or $R^1$ and $R^2$, working together, is phthaloyl group. This reaction is carried out by treating the benzoylacetic acid derivative with an equimolar or excess amount of the substituted pyrrolidine in a polar solvent such as acetonitrile, dimethyl sulfoxide (DMSO), N,N'-dimethylformamide (DMF), alcohol, dioxane, tetrahydrofuran (THF), water and their mixture at 0 °C to boiling point of the used solvent in the presence of a base catalyst. Examples of the base catalyst are inorganic base such as, e.g., sodium carbonate, sodium hydroxide, potassium hydroxide, sodium fluoride and potassium fluoride, organic base such as triethylamine, N,N-dimethylaniline, diazabicyclo-base and the like, or excess amount of the substituted pyrrolidine used for a base catalyst. The para-substituted compound (IV) is produced in excellent yield through this reaction. Moreover, it is very easy to eliminate the ortho-substituted isomer in the latter process, even if it is produced, because it can not be converted to a quinolonecarboxylic acid derivative which possesses a excellent nature for crystallizing.

The compound (IV) can be converted to a cyclopropylaminoacrylic acid derivative (V). Reacting the compound (IV) with ethyl orthoformate and acetic anhydride, or with dimethyl formamide dimethylacetal to form an condensed compound. The unpurified condensed product is allowed to treat with cyclopropylamine to give the compound (V). For example, the reaction is carried out by heating the compound (IV) with an equimolar or excess amount of ethyl orthoformate in acetic anhydride or with an equimolar or excess amount of dimethylformamide dimethylacetal in an inert solvent such as toluene, benzene and hexane, evaporating the reaction mixture, and then treating the resulting residue with an equimolar or exceess

amount of cyclopropylamine at 0 °C to room temperature in ethanol.

The compound (V) is cyclized in the presence of a base catalyst and, when a reaction product in which R is lower alkyl group and/or either $R^1$ or $R^2$ is the amino protective group is obtained, treating it with an acid or base, and/or reductive cleaving of it give the compound (I). Examples of the base catalyst are alkalimetal carbonate, alkalimetal hydroxide, alkalimetal hydride, alkalimetal alcoholate, tertialy amine and the like. For example, the cyclizing reaction is carried out in a solvent such as dioxane, THF, acetonitrile, alcohol, DMSO, DMF, water and their mixture in the presence of the above mentioned base catalyst (equimolar to 50 times molar excess) at 0 °C to boiling point of the used solvent.

When the product prepared from the above mentioned cyclizing reaction, has a lower acyl, alkoxycarbonyl and phthaloyl group as the amino protective group, it is converted to the compound (I) by the usual method, e.g., hydrolysis, reduction or acid treatment. When it has lower alkyl carboxylic ester group, it is hydrolyzed to the compound (I).

The synthetic intermediates (IV and V) are new compounds and important to complete the present invention. Therefore, this invention comprises the compounds of the formula (IV and V) as useful intermediates for the preparation of antibacterial agents.

The following examples will further illustrate the invention without, however, limiting it thereto.

Example 1. Ethyl 2-[4-(3-acetamido-1-pyrrolidinyl)-2,3-dichloro-5-fluorobenzoyl]acetate

A mixture of ethyl 2-(2,3-dichloro-4,5-difluorobenzoyl)acetate (1.77 g), 3-acetamidopyrrolidine (1.07 g), sodium bicarbonate (0.50 g) and dry acetonitrile (13 ml) was refluxed for 4 hours. After removing the solvent, to the residue was added water (30 ml) and extracted with dichloromethane (100 ml). The organic layer was washed with water and then dried over sodium sulfate. After removing the solvent, the resulting residue was purified by the silica gel column chromatography. The eluate of dichloromethane:acetone = 4:1 was concentrated under reduced pressure. The yellowish oil thus obtained was crystallized from ether to give the title compound as white powder (1.21 g), mp 93-96 °C.

Analysis (%) for $C_{17}H_{19}Cl_2FN_2O_4$, Calcd. (Found): C, 50.38 (50.46); H, 4.73 (4.82); N, 6.91 (6.84).

Example 2. Ethyl 2-[4-(3-acetamido-1-pyrrolidinyl)-3-chloro-2,5-difluorobenzoyl]acetate

A mixture of ethyl 2-(3-chloro-2,4,5-trifluorobenzoyl)acetate (1.00 g), 3-acetamidopyrrolidine (0.48 g) sodium bicarbonate (0.30 g) and dry acetonitrile (8 ml) was refluxed for 2.5 hours. Subsequent experiments were carried out as example 1. The desired compound was obtained (1.06 g), recrystallized from hexane-ethyl acetate to give the title compound as colorless needle, mp 102-103 °C.

Analysis (%) for $C_{17}H_{19}ClF_2N_2O_4$, Calcd. (Found): C, 52.52 (52.62); H, 4.93 (4.87); N, 7.20 (7.33).

Example 3. Ethyl 2-[4-(3-acetamido-1-pyrrolidinyl)-2,3-dichloro-5-fluorobenzoyl]-3-cyclopropylaminoacrylate

A mixture of ethyl 2-[4-(3-acetamido-1-pyrrolidinyl)-2,3-dichloro-5-fluorobenzoyl]acetate (1.20 g), dimethylformamide dimethylacetal (0.53 g) and toluene (5 ml) was stirred for 1.5 hours at 100-110 °C, and then concentrated under reduced pressure. The residue was dissolved in ethanol (7 ml), and a solution of cyclopropylamine (190 mg) in ethanol (1 ml) was added dropwise thereto under ice cooling, then stirred for 30 minutes at room temperature. After removing the solvent, the resulting residue was purified by the silica gel column chromatography. The eluate of dichloromethane:acetone = 4:1 was concentrated under reduced pressure to give the title compound as yellowish oil (1.37 g).

Example 4. Ethyl 2-[4-(3-acetamido-1-pyrrolidinyl)-3-chloro-2,5-difluorobenzoyl]-3-cyclopropylaminoacrylate

A mixture of ethyl 2-[4-(3-acetamido-1-pyrrolidinyl)-3-chloro-2,5-difluorobenzoyl]acetate (0.78 g), dimethylformamide dimethylacetal (0.40 ml) and toluene (5 ml) was stirred for an hour at 110-120 °C, and then dimethylformamide dimethylacetal (0.10 ml) was added thereto and stirred for 30 minutes at 110-120 °C. The subsequent processes were carried out as example 3. By using 0.13 g of cyclopropylamine, the title compound was obtained as yellowish green oil (0.77 g).

Example 5. Ethyl 7-(3-acetamido-1-pyrrolidinyl)-8-chloro-1-cyclopropyl-6-fluoro-1,4-dihydro-4-oxo-3-quinoline carboxylate.

a) A solution of ethyl 2-[4-(3-acetamido-1-pyrrolidinyl)-2,3-dichloro-5-fluorobenzoyl]-3-cyclopropylaminoacrylate (400 mg) in dry dioxane (1 ml) was added dropwise to a suspension of 55 % sodium hydride (190 mg) in dry dioxane (3 ml) under ice cooling, and then refluxed for an hour. The reaction mixture was poured into ice water (20 ml), the precipitate was collected by filtration, washed with water and n-hexane successively, and recrystallized from dichloromethane-methanol to give the title compound as white powder (123 mg), mp 206-208 °C.

Analysis (%) for $C_{21}H_{23}ClFN_3O_4$, Calcd. (Found): C, 57.87 (57.87); H, 5.32 (5.38); N, 9.64 (9.56).

b) A solution of ethyl 2-[4-(3-acetamido-1-pyrrolidinyl)-3-chloro-2,5-difluorobenzoyl]-3-cyclopropylaminoacrylate (0.69 g) in dry dioxane (2 ml) was added dropwise to a suspension of 55 % sodium hydride (70 mg) in dry dioxane (5 ml), stirred for an hour at 90-100 °C, and then 55 % sodium hydride (300 mg) was added thereto and stirred for 40 minutes at 90-100 °C. The reaction mixture was poured into ice water, the precipitate was collected by filtration, washed with water and n-hexane successively to give the title compound as grayish needles (0.40 g), mp 210-212 °C.

Example 6. 7-(3-Amino-1-pyrrolidinyl)-8-chloro-1-cyclopropyl-6-fluoro-1,4-dihydro-4-oxo-3-quinolinecarboxylic acid

A mixture of ethyl 7-(3-acetamido-1-pyrrolidinyl)-8-chloro-1-cyclopropyl-6-fluoro-1,4-dihydro-4-oxo-3-quinolinecarboxylate (400 mg) and 2 N sodium hydroxide solution (20 ml) was refluxed for 4 hours, neutralized with acetic acid under heating. After cooling, the precipitate was collected by filtration and dis solved in acetic acid-water (0.5 ml-20 ml). After removing the insoluble compound, the filtrate was adjusted to pH 10.0 by adding 50 % sodium hydroxide solution, and then neutralized with acetic acid. The precipitate was collected by filtration, washed with water, methanol and ether successively to give the title compound as pale yellow powder (0.205 g), mp 241-245 °C (dec.).

Analysis (%) for $C_{17}H_{17}ClFN_3O_3 \bullet 9/5\ H_2O$, Calcd. (Found): C, 51.27 (51.17); H, 5.21 (5.07); N, 10.55 (10.34).

Example 7. Ethyl 2-[2,3-dichloro-4-(3-ethoxycarbonylamino-1-pyrrolidinyl)-5-fluorobenzoyl]acetate

A mixture of ethyl 2-(2,3-dichloro-4,5-difluorobenzoyl)acetate (3.0 g), 3-ethoxycarbonylaminopyrrolidine (1.90 g), potassium fluoride (1.16 g) and dry dimethyl sulfoxide (20 ml) was stirred for 3 hours at 50 °C, and then 3-ethoxycarbonylaminopyrrolidine (0.32 g) and potassium fluoride (0.29 g) was added thereto and stirred for an hour at 50 °C. After removing the solvent, to the residue was added water (20 ml) and extracted with chloroform (50 ml). The organic layer was washed with water and then dried over sodium sulfate. After concentrating under reduced pressure, the resulting residue was purified by the silica gel column chromatography. The eluate of chloroform:methanol = 40:1 was concentrated under reduced pressure. Crystallization was crystallized from n-hexane-ether gave the title compound as white powder (2.39 g), mp 80-82 °C.

Analysis (%) for $C_{18}H_{21}Cl_2FN_2O_5$, Calcd. (Found): C, 49.67 (49.71); H, 4.86 (4.85); N, 6.44 (6.44).

Example 8. Ethyl 2-[2,3-dichloro-4-(3-ethoxycarbonylamino-1-pyrrolidinyl)-5-fluorobenzoyl]-3-cyclopropylaminoacrylate

A mixture of ethyl 2-[2,3-dichloro-4-(3-ethoxycarbonylamino1-pyrrolidinyl)-5-fluorobenzoyl]acetate (2.20 g), dimethylform-amide dimethylacetatal (0.90 g) and dry toluene (10 ml) was stirred for an hour at 100-110 °C, and then dimethylformamide dimethylacetal (0.20 g) was added thereto and stirred for an hour at 100-110 °C, concentrated under reduced pressure. The residue was dissolved in ethanol (13 ml), and a solution of cyclopropylamine (0.32 g) in ethanol (2 ml) was added dropwise thereto under ice cooling, then stirred for 30 minutes at room temperature. After removing the solvent, the resulting residue was purified by the silica gel chromatography. The eluate of dichloromethane was concentrated under reduced pressure to give the title compound as yellowish oil (2.48 g).

Example 9. Ethyl 8-chloro-1-cyclopropyl-7-(3-ethoxycarbonylamino-1-pyrrolidinyl)-6-fluoro-1,4-dihydro-4-oxo-3-quinolinecarboxylate

A solution of ethyl 2-[2,3-dichloro-4-(3-ethoxycarbonylamino-1-pyrrolidinyl)-5-fluorobenzoyl]-3-cyclopropylaminoacrylate (1.30 g) in dry dioxane (3 ml) was added dropwise to a suspension of 60 % sodium hydride (0.52 g) in dry dioxane (9 ml) under ice cooling, the refluxed for an hour. The reaction mixture was poured into ice water (100 ml), the precipitate was collected by filtration and dissolved in dichloromethane-methanol. The solution was effectively treated by activated charcoal powder. After removing the solvent, the reddish oil as residue was crystallized from ether to give the title compound as pale brownish powder (0.52 g), mp 106-109 °C.

Analysis (%) for $C_{22}H_{25}ClFN_3O_5$, Calcd. (Found): C, 56.71 (56.23); H, 5.41 (5.32); N, 9.02 (8.82).

Example 10. 7-(3-Amino-1-pyrrolidinyl)-8-chloro-1-cyclopropyl-6-fluoro-1,4-dihydro-4-oxo-3-quinolinecarboxylic acid

A mixture of ethyl 8-chloro-1-cyclopropyl-7-(3-ethoxycarbonylamino-1-pyrrolidinyl)-6-fluoro-1,4-dihydro-4-oxo-3-quinolinecarboxylate (400 mg) and 2 N sodium hydroxide solution (20 ml) was refluxed for 2 hours, neutralized with acetic acid under heating. After cooling, the precipitate was collected by filtration and dissolved in acetic acid-water (0.3 ml-3ml). After removing the insoluble compound, the filtrate was adjusted to pH 10.0 by adding 50% sodium hydroxide solution, and then neutralized with acetic acid. The precipitate was collected by filtration, washed with water, methanol and ether successively to give the title compound as pale reddish powder (0.204 g), mp 235-240 °C (dec.).

Analysis (%) for $C_{17}H_{17}ClFN_3O_3 \bullet H_2O$, Calcd. (Found): C, 53.20 (53.52); H, 4.99 (5.22); N, 10.95 (10.72).

## Claims

1. A process for the preparation of a compound of the formula (I);

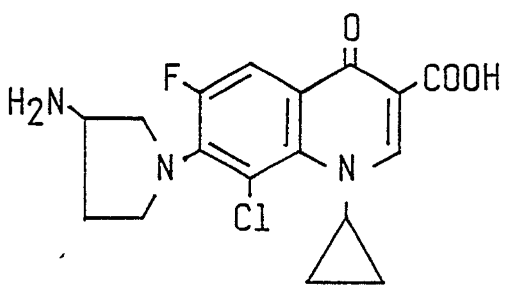

(I)

which comprises:

(a) reacting a benzoylacetic acid derivative of the formula (II);

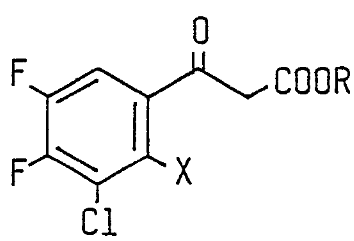

(II)

wherein R is hydrogen atom or lower alkyl group and X is halogen atom, with pyrrolidine derivative of the formula (III);

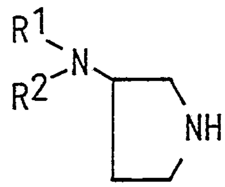

(III)

wherein $R^1$ and $R^2$ each independently are hydrogen atom or a protective group for the amino group, or $R^1$ together with $R^2$ is a protective group, to give a compound of the formula (IV);

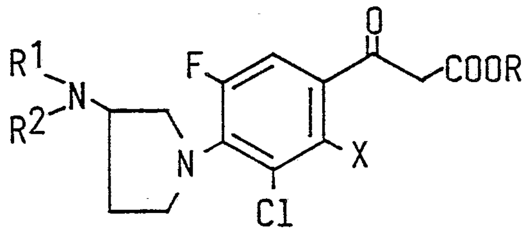

(IV)

wherein R, $R^1$, $R^2$ and X are as defined above,

(b) reacting the compound of the formula (IV) with ethyl ortho formate and acetic anhydride, or with dimethylformamide dimethylacetal to form a condensed compound and then treating the condensed compound with cyclopropylamine to give a acrylic acid derivative of the formula(V);

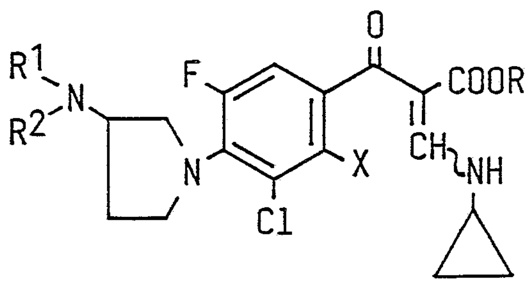

(V)

wherein R, $R^1$, $R^2$ and X are as defined above,

(c) cyclizing the acrylic acid derivative (V) in the presence of a base catalyst and when a reaction product in which R is lower alkyl group and/or either $R^1$ or $R^2$ is the amino protective group is obtained, treating it with an acid or base, and/or reductively cleaving it.

2. A process according to claim 1, wherein $R^1$ and $R^2$ defined each independently are hydrogen atom, lower alkyl group or alkoxycarbonyl group, or $R^1$ together with $R^2$ is phthaloyl group.

3. A benzoylacetic acid derivative of the formula (IV);

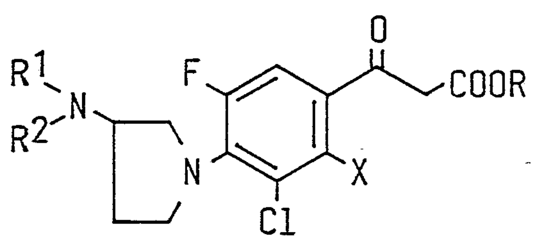

(IV)

wherein R is hydrogen atom or lower alkyl group, X is halogen atom, $R^1$ and $R^2$ each defined independently are hydrogen atom or a protective group for the amino group, or $R^1$ together with $R^2$ is a protective group.

4. A benzoylacrylic acid derivative of the formula (V);

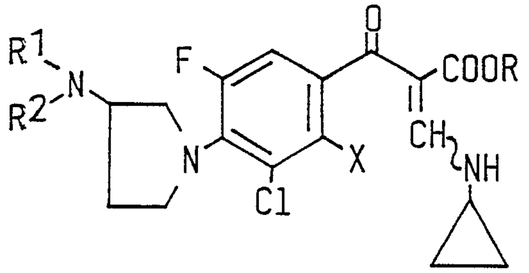

(V)

wherein R is hydrogen atom or lower alkyl group, X is halogen atom, $R^1$ and $R^2$ each independently are hydrogen atom or a protective group for the amino group, or $R^1$ together with $R^2$ is a protective group.

Claims for the following contracting state: ES

1. A process for the preparation of a compound of the formula (I);

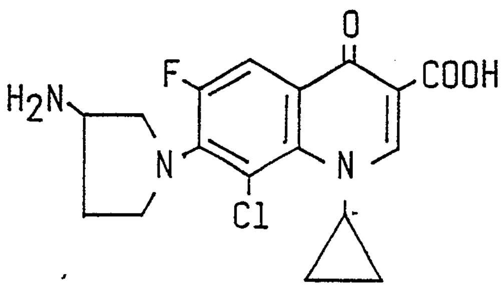

(I)

which comprises:

(a) reacting a benzoylacetic acid derivative of the formula (II);

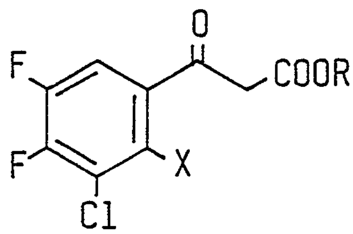

(II)

wherein R is hydrogen atom or lower alkyl group and X is halogen atom, with pyrrolidine derivative of the formula (III);

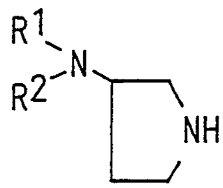

(III)

wherein $R^1$ and $R^2$ each independently are hydrogen atom or a protective group for the amino group, or $R^1$ together with $R^2$ is a protective group, to give a compound of the formula (IV);

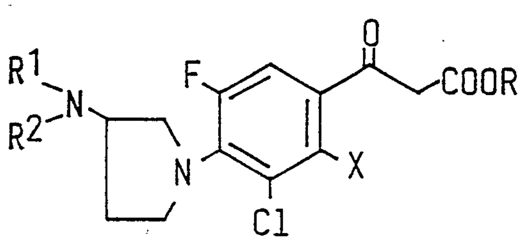

(IV)

wherein R, $R^1$, $R^2$ and X are as defined above,

(b) reacting the compound of the formula (IV) with ethyl ortho formate and acetic anhydride, or with dimethylformamide dimethylacetal to form a condensed compound and then treating the condensed compound with cyclopropylamine to give a acrylic acid derivative of the formula (V);

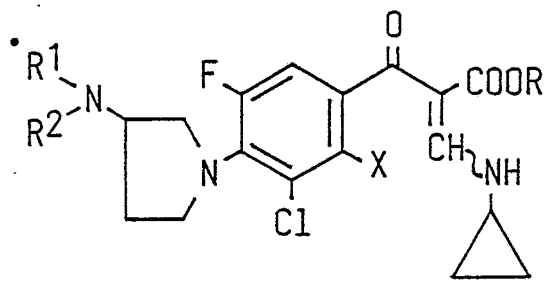

(V)

wherein R, $R^1$, $R^2$ and X are as defined above,

(c) cyclizing the acrylic acid derivative (V) in the presence of a base catalyst and when a reaction product in which R is lower alkyl group and/or either $R^1$ or $R^2$ is the amino protective group is obtained, treating it with an acid or base, and/or reductively cleaving it.

2. A process according to claim 1, wherein $R^1$ and $R^2$ defined each independently are hydrogen atom, lower alkyl group or alkoxycarbonyl group, or $R^1$ together with $R^2$ is phthaloyl group.

European Patent Office

# EUROPEAN SEARCH REPORT

Application number: EP 88100683.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | EP - A1 - 0 195 316 (KYORIN PHARMACEUTICAL)<br>* Formula I, table pages 9,10 *<br>-- | 1 | C 07 D 401/04<br>C 07 D 207/14 |
| Y | EP - A1 - 0 183 129 (KYORIN PHARMACEUTICAL)<br>* Examples 22,35 *<br>-- | 1 | |
| A | CHEMICAL ABSTRACTS, vol. 98, no. 24, June 13, 1983, Columbus, Ohio, USA<br>DANIELS, JOHN Y. "New malonic acid dyes for PET and PET/cotton blends" page 71, column 1, abstract-no. 199 813u<br>& Am. Dyest. Rep. 1983, 72(1), 26, 28-34<br>-- | 3 | |
| A | EP - A2 - 0 003 663 (AMERICAN CYANAMID)<br>* Totality *<br>---- | 3,4 | |

TECHNICAL FIELDS SEARCHED (Int. Cl.4)

C 07 D 401/00
C 07 D 207/00

The present search report has been drawn up for all claims

| Place of search | Date of completion of the search | Examiner |
|---|---|---|
| VIENNA | 22-03-1988 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document